(19) Europäisches Patentamt
European Patent Office
Office européen des brevets

(11) Veröffentlichungsnummer: **0 159 519**
**A2**

(12) # EUROPÄISCHE PATENTANMELDUNG

(21) Anmeldenummer: **85102913.2**

(22) Anmeldetag: **14.03.85**

(51) Int. Cl.[4]: **A 61 K 31/095,** A 61 K 31/10, A 61 K 31/195, A 61 K 31/22

(30) Priorität: **29.03.84 DE 3411617**

(43) Veröffentlichungstag der Anmeldung: **30.10.85 Patentblatt 85/44**

(84) Benannte Vertragsstaaten: **AT BE CH DE FR GB IT LI LU NL SE**

(71) Anmelder: **Asta-Werke Aktiengesellschaft Chemische Fabrik, Artur-Ladebeck-Strasse 128 - 152, D-4800 Bielefeld 14 (DE)**

(72) Erfinder: **Stekar, Jurij, Dr., Asta-Strasse 29, D-4800 Bielefeld 13 (DE)**
Erfinder: **Hilgard, Peter, Dr., Detmolderstrasse 16, D-4800 Bielefeld 1 (DE)**

(74) Vertreter: **Nowak, Gerhard, DEGUSSA AG Fachbereich Patente Postfach 1345, D-6450 Hanau 1 (DE)**

(54) **Verwendung von Thioverbindungen zur Verhinderung des durch Cytostatika verursachten Haarausfalls.**

(57) Der durch Cytostatika verursachte Haarausfall kann durch Applikation von Verbindungen der allgemeinen Formel

$$Alk_1–S–Alk_2$$

worin $Alk_1$ einen Phenylrest oder einen gegebenenfalls durch Guanidino, Hydroxy, Mercapto, Sulfo, Amino, Carboxy, Phenylamino oder Cyclohexylamino substituierten $C_1$–$C_6$-Alkylrest darstellt und $Alk_2$ Wasserstoff ist oder S–$Alk_1$ bedeutet oder worin beide Reste $Alk_1$ und $Alk_2$ zusammen mit dem Schwefelatom einen gesättigten 5-Ring aus $CH_2$-Gliedern bilden, wobei ein Ringglied auch eine NH-Gruppe oder eine CO-Gruppe sein kann oder worin $Alk_1$ Wasserstoff oder ein gegebenenfalls durch Amino substituierter $C_1$–$C_8$-Alkylrest ist und $Alk_2$ die Gruppe -$SO_3H$ oder die Gruppe C(Y)-$NR_8R_9$ bedeutet, worin Y die Gruppe $=R_{10}$ oder ein Schwefelatom ist und die Reste $R_8$, $R_9$ und $R_{10}$ Wasserstoff oder $C_1$–$C_6$-Alkylreste sind oder durch folgende Verbindungen: 2-Mercapto-ethylphosphorsäure, Amidothiophosphorsäure, S-(2-Aminoethyl)-thiophosphorsäure, S–2–(3-Aminopropylamino)-ethyl-thiophosphorsäure, Guanylthioharnstoff, Phenylthioharnstoff, α-Liponsäure, α-Liponsäureamid beziehungsweise deren Salzen verhindert werden.

EP 0 159 519 A2

ASTA-Werke Aktiengesellschaft, Chemische Fabrik
Artur-Ladebeck-Straße 128-152, 4800 Bielefeld 14

# Verwendung von Thioverbindungen zur Verhinderung des durch Cytostatika verursachten Haarausfalls

Eine stark belastende Nebenwirkung der cytostatischen Chemotherapie ist der durch die Cytostatika verursachte Haarausfall (Alopezie). Es wird daher seit Jahren nach Möglichkeiten gesucht, diese Komplikation bei der cytostatischen Chemotherapie zu verhindern oder zumindest zu vermindern. Die Wirkung der zu diesem Zweck eingesetzten therapeutischen Massnahmen (beispielsweise Kältekappen) ist jedoch nach wie vor unbefriedigend.
Eine medikamentöse Verhinderung der Cytostatika-Alopezie wäre daher für die Patienten eine grosse Erleichterung und würde einen bedeutenden therapeutischen Fortschritt darstellen.

Es wurde nun überraschend gefunden, daß durch Verwendung der in Anspruch 1 genannten chemischen Verbindungen beziehungsweise mit einer Kombination solcher Verbindungen der durch die Chemotherapie mit Cytostatika verursachte Haarausfall verhindert beziehungsweise stark vermindert werden kann.

Die Erfindung betrifft die durch die Patentansprüche definierten Gegenstände.

...

Für die erfindungsgemässe Verwendung kommen insbesondere folgende Verbindungen in Betracht:

1. Verbindungen der Formel Alkyl-SH und deren Disulfide, worin Alkyl ein $C_1$-$C_6$-Alkylrest, insbesondere ein $C_1$-$C_4$-Alkylrest, vorzugsweise ein $C_1$-$C_2$-Alkylrest ist, welcher durch eine Sulfogruppe, eine Carboxygruppe, eine Carb-$C_1$-$C_4$-alkoxygruppe, eine Carboxymethylaminogruppe oder eine Carboxymethylaminocarbonylgruppe substituiert ist, wie zum Beispiel 2-Mercapto-essigsäure, 2-Mercapto-propionsäure, 3-Mercapto-propionsäure, 3-Mercapto-2-methyl-propionsäure, 2-Mercapto-buttersäure, 2-Mercapto-buttersäureethylester (2-Mercapto-ethylamino)-essigsäure, N-(2-mercapto-propionyl)-glycin, 2-Mercapto-ethansulfonsäure (Na-Salz), 3-Mercapto-n-propansulfonsäure (Na-Salz), 3-Mercapto-2-methyl-n-propansulfonsäure (Na-Salz).

2. Verbindungen der Formel Alkyl-SH und deren Disulfide, worin Alkyl ein $C_1$-$C_6$-Alkylrest, insbesondere ein $C_1$-$C_4$-Alkylrest, vorzugsweise ein $C_1$-$C_3$-Alkylrest ist, welcher durch gleiche oder verschiedene Reste $R'_1$, $R'_2$ und $R'_3$ substituiert ist, wobei $R'_1$ und $R'_2$ eine Hydroxygruppe, eine Mercaptogruppe, eine Aminogruppe eine $C_2$-$C_6$-Alkanoylaminogruppe, eine Sulfogruppe, eine Carboxygruppe oder eine $C_1$-$C_4$-Alkoxycarbonylgruppe bedeuten und $R'_3$ Wasserstoff, eine Hydroxygruppe oder eine Mercaptogruppe ist. Falls in der Formel Alkyl-SH der Alkylrest 2 Substituenten enthält, ist der eine Substituent insbesondere eine Carboxygruppe oder eine $C_1$-$C_4$-Alkoxycarbonylgruppe (vorzugsweise in $\omega$-Stellung zur SH-Gruppe) und der andere Substituent insbesondere eine Aminogruppe oder eine $C_2$-$C_6$-Alkanoylaminogruppe (vorzugsweise in

...

Nachbarstellung zur Carbonyl- oder Alkoxycarbonylgruppe, wenn letztere in ω-Stellung bezüglich der SH-Gruppe stehen). Beispiele hierfür sind Cystein, Cysteinmethylester, Cysteinethylester, 2,3-Dihydroxy-1,4-dimercapto-butan, 2,3-Dimercaptopropanol, 2,3-Dimercapto-bernsteinsäure, 2-Amino-4-mercapto-buttersäure, 2,3-Dimercapto-n-propansulfonsäure (Na-Salz), N-Acetyl-cystein.

3. Verbindungen der Formel Alkyl-SH und deren Disulfide, worin Alkyl ein $C_1$-$C_6$-Alkylrest, insbesondere ein $C_1$-$C_4$-Alkylrest, vorzugsweise ein $C_1$-$C_2$-Alkylrest ist, welcher durch eine Phenylaminogruppe (gegebenenfalls im Phenylkern substituiert durch Amino) oder eine $C_5$-$C_7$-Cycloalkylaminogruppe substituiert ist, wobei eine $CH_2$-Einheit des Cycloalkylrestes auch durch ein Stickstoff- oder Sauerstoffatom ersetzt sein kann, wie zum Beispiel N-Phenyl-cysteamin, N-Piperidyl-(4)-cysteamin.

4. Verbindungen der Formel Alkyl-SH und deren Disulfide, worin Alkyl ein $C_1$-$C_6$-Alkylrest, insbesondere ein $C_1$-$C_4$-Alkylrest, vorzugsweise ein $C_1$-$C_2$-Alkylrest ist, welcher einen Guanidinorest enthält, wobei die freie $NH_2$-Gruppe und/oder die freie Iminogruppe des Guanidinorestes auch durch $C_1$-$C_4$-Alkylreste (insgesamt bis zu 3 Alkylresten) substituiert sein können und deren Säureadditionssalze, wie zum Beispiel (2-Mercaptoethyl)-guanidin, (3-Mercapto-propyl)-guanidin, N-(2-Mercaptoethyl)-N'-methyl-guanidin.

5. Thiophenole und deren Disulfide, die im Phenylring (vorzugsweise in 2- oder 4-Stellung) durch eine Aminogruppe substituiert sind, wie zum Beispiel o-Aminothiophenol.

...

6. Verbindungen der Formel Alkyl-S-$SO_3H$ und deren Salze (insbesondere Alkalisalze), worin Alkyl ein $C_1$-$C_6$-Alkylrest, insbesondere ein $C_1$-$C_4$-Alkylrest, vorzugsweise ein $C_1$-$C_2$-Alkylrest ist, der eine Aminogruppe (vorzugsweise in 2-Stellung) enthält, wie zum Beispiel S-(2-Amino-ethyl)-thioschwefelsäure (zum Beispiel in Form des Natriumsalzes).

7. Verbindungen der Formel $R'_4$-S-C(Y)-$NH_2$, worin Y die Gruppe =NH oder ein Schwefelatom ist und $R'_4$ Wasserstoff oder ein $C_2$-$C_6$-Alkylrest, insbesondere $C_2$-$C_4$-Alkylrest, vorzugsweise $C_2$-$C_3$-Alkylrest ist, welcher eine Aminogruppe (vorzugsweise in 2-Stellung) enthält und die $NH_2$-Gruppe und/oder NH-Gruppe des Restes -C(=NH)-$NH_2$ insgesamt auch einen, zwei oder drei $C_1$-$C_4$-Alkylreste enthalten können, wobei ausserdem die NH-Gruppe oder die $NH_2$-Gruppe auch einen Phenylrest oder einen Pyridylrest enthalten kann und deren Säureadditionssalze beziehungsweise deren Salze mit Kationen (Alkalimetalle, $NH_4$), falls es sich um Carbaminsäurederivate handelt wie zum Beispiel S-(2-Amino-ethyl)-isothioharnstoff, S-(2-Amino-ethyl)-N-methyl-isothioharnstoff, S-(2-Amino-ethyl)-N,N'-dimethyl-isothioharnstoff, S-(2-Amino-ethyl)-N,N-dimethyl-isothioharnstoff, Thioharnstoff, 1-Methyl-3-isopropyl-thioharnstoff, 1-Isopropyl-3(2-pyridyl)-thioharnstoff, Phenylthioharnstoff, Dithiocarbaminsäure (zum Beispiel als Ammoniumsalze), Diethyldithiocarbaminsäure (zum Beispiel als Natriumsalz). Falls $R'_4$ Wasserstoff ist, können diese Verbindungen natürlich auch in der tautomeren Form $CS(NH_2)_2$ vorliegen.

...

8. Verbindungen der Formel

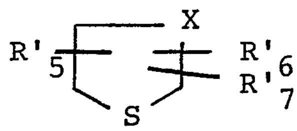

worin X eine $CH_2$-Gruppe oder eine NH-Gruppe ist, eine $CH_2$-Einheit des 5-Ringes in Nachbarstellung zum Schwefel auch eine CO-Gruppe sein kann und die Reste $R'_5$ $R'_6$ und $R'_7$ gleich oder verschieden sind und $C_1$-$C_4$-Alkylgruppen, Carboxygruppen, eine Aminogruppe oder eine $C_2$-$C_6$-Alkanoylaminogruppe darstellen, wie zum Beispiel Thioprolin, 2,2-Dimethyl-thiazolidin-4-carbonsäure, Homocysteinthiolacton, N-Acetyl-homocysteinthiolacton.
Insbesondere handelt es sich hierbei um Thiazolidine (X=NH), die gegebenenfalls in 2-Stellung eine oder zwei $C_1$-$C_4$-Alkylreste und in 4-Stellung eine Carboxygruppe haben oder um 5-Ring-Thiolactone ($X=CH_2$), die in 3-Stellung eine Amino- oder $C_2$-$C_6$-Alkanoylaminogruppe haben.

...

Die in der Formel I vorkommenden Alkylgruppen, Monoalkylaminogruppen, Dialkylaminogruppen, Alkanoylaminogruppen können gerade oder verzweigt sein; Die vorkommenden Alkylgruppen bestehen im allgemeinen aus 1-4 Kohlenstoffatomen, vorzugsweise 1, 2 oder 3 Kohlenstoffatomen.

Die erfindungsgemäßen Thioverbindungen können vorteilhaft in Form ihrer Salze mit einer organischen oder anorganischen Säure verwendet werden, wenn diese Verbindungen mindestens eine primäre Aminogruppe umfassen. Wenn jedoch die primäre Aminogruppe mit einer im Molekül anwesenden Carboxylgruppe ein inneres Salz bildet, so kann man das Salz der Verbindung nur unter Verwendung einer starken Säure, die beispielsweise eine anorganische Säure oder p-Toluolsulfonsäure oder Trichloressigsäure sein kann, erhalten.

Die Säuren, die zur Salzbildung an der Aminfunktion verwendet werden können, sind im allgemeinen ausgewählt unter Chlorwasserstoffsäure, Bromwasserstoffsäure, Schwefelsäure, Dihydrogenphosphorsäure, p-Toluolsulfonsäure, Dihydrozimtsäure, Zimtsäure, Mandelsäure, Salicylsäure, Tropasäure, Oxalsäure, Apfelsäure, Weinsäure, Bernsteinsäure, Essigsäure, Milchsäure, 3-Hydroxybuttersäure, Fumarsäure, Undecylensäure, Phenylessigsäure, Monochloressigsäure, Trichloressigsäure, Glycolsäure, Nicotinsäure, p-Aminobenzoesäure, Glutaminsäure, Asparaginsäure, Zitronensäure, Panthotensäure und Krotonsäure.

Falls die erfindungsgemässen Thioverbindungen saure Gruppen enthalten (zum Beispiel Dithiocarbaminsäurederivate, Thioschwefelsäurederivate) können auch

...

entsprechende Salze mit physiologisch unbedenklichen basischen Verbindungen verwendet werden. Als Kationen kommen hierbei zum Beispiel in Frage: Natrium und Ammonium.

...

Die erfindungsgemässen Thioverbindungen wirken prophylaktisch bei der Alopezie, welche beispielsweise durch folgende Cytostatika induziert wird: Oxazaphosphorine (zum Beispiel Cyclophosphamid, Ifosfamid, Trofosfamid, Sufosfamid; siehe Belgische Patente 554 888, 700 711, Deutsche Offenlegungsschrift 2 107 936), aktivierte stabilisierte Oxazaphosphorine (siehe Deutsche Offenlegungsschrift 3 133 309, Belgisches Patent 892 589, Europäische Patentanmeldung 0 083 439, Deutsche Patentanmeldung P 34 07 585.2), Adriamycin, Cisplatin, Podophyllotoxin-Derivate, zum Beispiel Mitopodozid, Etoposid (4'-Demethyl-epipodophyllotoxin-9-(4,6-0-ethyliden-ß-D-glucopyranosid) oder Podophyllsäure-(2-ethyl-hydrazid).

Nitrosoharnstoffe (zum Beispiel N,N'-Bis-(2-chlorethyl)-N-nitrosoharnstoff, N-(2-Chlor-ethyl)-N'-cyclohexyl-N-nitroso-harnstoff, N-(2-Chlor-ethyl)-N'-(4-methyl-cyclohexyl)-N-nitroso-harnstoff, Belgisches Patent 855 043).

Die Applikation der erfindungsgemässen Thioverbindungen erfolgt vorzugsweise in Form von Lösungen dieser Verbindungen und zwar perkutan. Gegebenenfalls ist beispielsweise auch eine orale oder parenterale Applikation möglich.
Als Lösungsmittel für die erfindungsgemässen Schwefelverbindungen kommen beispielsweise in Betracht: Wasser sowie andere physiologisch verträgliche mit Wasser mischbare organische Lösungsmittel, wie zum Beispiel Ethanol, Isopropanol, Glycerin, Polyalkylenglykole, Dimethylformamid, Dimethylacetamid sowie Mischungen dieser Mittel.
Als Lösungsmittelmischungen kommen zum Beispiel in Betracht: Ethanol-Wassermischungen (mit 10-50 Gewichtsteilen Ethanol), Glycerin-Wassermischungen (mit 40 Gewichtsteilen Glycerin).

...

Die erfindungsgemäßen Verbindungen sind zur Herstellung pharmazeutischer Zusammensetzungen und Zubereitungen geeignet. Die pharmazeutischen Zusammensetzungen beziehungsweise Arzneimittel enthalten als Wirkstoff einen oder mehrere der erfindungsgemäßen Verbindungen, gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.
Als derartige Träger- und Hilfsstoffe kommen zum Beispiel solche Stoffe in Frage, die in folgenden Literaturstellen als Hilfsstoffe für Pharmazie, Kosmetik und angrenzende Gebiete empfohlen beziehungsweise angegeben sind: Ullmanns Encyklopädie der technischen Chemie, Band 4 (1953), Seite 1 bis 39; Journal of Pharmaceutical Sciences, Band 52 (1963), Seite 918 u.ff., H.v.Czetsch-Lindenwald, Hilfsstoffe für Pharmazie und angrenzende Gebiete; Pharm. Ind., Heft 2, 1961, Seite 72 u.ff.; Dr. H. P. Fiedler, Lexikon der Hilfsstoffe für Pharmazie, Kosmetik und angrenzende Gebiete Cantor KG. Aulendorf in Württemberg 1971.

Beispiele hierfür sind Gelatine, natürliche Zucker wie Rohrzucker oder Milchzucker, Lecithin, Pektin, Stärke (zum Beispiel Maisstärke), Gelatine, Gummiarabikumi, Alginsäure, Tylose, Talkum, Lycopodium, Kieselsäure (zum Beispiel kolloidale), Cellulose, Cellulosederivate (zum Beispiel Celluloseether, bei denen die Cellulose-Hydroxygruppen teilweise mit niederen gesättigten aliphatischen Alkoholen und/oder niederen gesättigten aliphatischen Oxyalkoholen verethert sind, zum Beispiel

...

Methyloxypropylcellulose, Methylcellulose, Hydroxypropylmethylcellulose), Stearate, Magnesium- und Calciumsalze von Fettsäuren mit 12 bis 22 C-Atomen, insbesondere der gesättigten (zum Beispiel Stearate), Emulgatoren, Öle und Fette, insbesondere pflanzliche (zum Beispiel Erdnussöl, Rhizinusöl, Olivenöl, Sesamöl, Baumwollsaatöl, Maisöl, Weizenkeimöl, Sonnenblumensamenöl, Kabeljau-Leberöl, Mono-, Di- und Triglyceride von gesättigten Fettsäuren $C_{12}H_{24}O_2$ bis $C_{18}H_{36}O_2$ und deren Gemische), pharmazeutisch verträgliche ein- oder mehrwertige Alkohole und Polyglykole wie Polyethylenglykole sowie Derivate hiervon, Ester von aliphatischen gesättigten oder ungesättigten Fettsäuren (2 bis 22 C-Atome, insbesondere 10 bis 18 C-Atome) mit einwertigen aliphatischen Alkoholen (1 bis 20 C-Atome) oder mehrwertigen Alkoholen wie Glykolen, Glycerin, Diethylenglykol, Pentaerythrit, Sorbit, Mannit und so weiter, die gegebenenfalls auch verethert sein können, Benzylbenzoat, Dioxolane, Glyzerinformale, Tetrahydrofurfurylalkohol, Polyglykolether mit $C_1$-$C_{12}$-Alkoholen, Dimethylacetamid, Lactamide, Lactate, Ethylcarbonate, Silicone (insbesondere mittelviskose Dimethylpolysiloxane), Calciumcarbonat, Natriumcarbonat, Calciumphosphat, Natriumphosphat, Magnesiumcarbonat und ähnliche.

Als weitere Hilfsstoffe kommen auch Stoffe in Frage, die den Zerfall bewirken (sogenannte Sprengmittel) wie: quervernetztes Polyvinylpyrrolidon, Natriumcarboxymethylstärke, Natriumcarboxymethylcellulose oder mikrokristalline Cellulose. Ebenfalls können bekannte Hüllstoffe verwendet werden wie zum Beispiel: Polyacrylsäureester, Celluloseether und ähnliche.

Zur Herstellung von Lösungen kommen beispielsweise Wasser oder physiologisch verträgliche organische Lösungsmittel

...

in Frage, wie zum Beispiel Ethanol, 1,2-Propylenglykol, Polyglykole und deren Derivate, Dimethylsulfoxyd, Dimethylformamid, Dimethylacetamid, Fettalkohole, Trigylceride, Partialester des Glycerins, Paraffine und ähnliche. Für injizierbare Lösungen oder Suspensionen kommen zum Beispiel nicht-toxische parenteral verträgliche Verdünnungsmittel oder Lösungsmittel in Frage, wie zum Beispiel: 1,3-Butandiol, Wasser, Ringer's Lösung, isotonische Kochsalzlösung oder auch gehärtete öle einschließlich synthetischer Mono- oder Diglyceride oder Fettsäuren wie Oleinsäure.

Bei der Herstellung der Zubereitungen können bekannte und übliche Lösungsvermittler, beziehungsweise Emulgatoren, verwendet werden. Als Lösungsvermittler und Emulgatoren kommen beispielsweise in Frage: Polyvinylpyrrolidon, Sorbitanfettsäureester wie Sorbitantrioleat, Phosphatide, wie Lecithin, Acacia, Tragacanth, polyoxyethyliertes Sorbitanmonooleat und andere ethoxylierte Fettsäureester des Sorbitan, polyoxyethylierte Fette, polyoxyethylierte Oleotriglyceride, linolisierte Oleotriglyceride, Polyethylenoxyd-Kondensationsprodukte von Fettalkoholen, Alkylphenolen oder Fettsäuren oder auch 1-Methyl-3-(2-hydroxyethyl)-imidazolidon-(2). Polyoxyethyliert bedeutet hierbei, daß die betreffenden Stoffe Polyoxyethylenketten enthalten, deren Polymerisationsgrad im allgemeinen zwischen 2 bis 40 und insbesondere zwischen 10 bis 20 liegt.
Solche polyoxyethylierten Stoffe können beispielsweise durch Umsetzung von hydroxylgruppenhaltigen Verbindungen (beispielsweise Mono- oder Diglyceride oder ungesättigte Verbindungen wie zum Beispiel solchen die ölsäurereste enthalten) mit Ethylenoxyd erhalten werden (zum Beispiel 40 Mol Ethylenoxyd pro Mol Glycerid).
Beispiele für Oleotriglyceride sind Olivenöl, Erdnussöl, Rhizinusöl, Sesamöl, Baumwollsaatöl, Maisöl (siehe auch

...

Dr. H.P. Fiedler "Lexikon der Hilfsstoffe für Pharmazie, Kosmetik und angrenzende Gebiete" 1971, Seite 191 bis 195)

Darüberhinaus ist der Zusatz von Konservierungsmitteln, Stabilisatoren, Puffersubstanzen, zum Beispiel Calciumhydrogenphosphat, kolloidales Aluminiumhydroxyd, Süßmitteln, Farbstoffen, Antioxydantien und Komplexbildnern (zum Beispiel Ethylendiaminotetraessigsäure) und dergleichen möglich. Gegebenenfalls ist zur Stabilisierung des Wirkstoffmoleküls mit physiologisch verträglichen Säuren oder Puffern auf einen pH-Bereich von ca. 3 bis 7 einzustellen. Im allgemeinen wird ein möglichst neutraler bis schwach saurer (bis pH 5) pH-Wert bevorzugt.

Als Antioxydantien kommen beispielsweise Natriummetabisulfit, Ascorbinsäure, Gallussäure, Gallussäure-alkylester, Butylhydroxyanisol, Nordihydroguajaretsäure, Tocopherole sowie Tocopherole + Synergisten (Stoffe die Schwermetalle durch Komplexbildung binden, beispielsweise Lecithin, Ascorbinsäure, Phosphorsäure) zur Anwendung. Der Zusatz der Synergisten steigert die antioxygene Wirkung der Tocopherole erheblich.
Als Konservierungsmittel kommen beispielsweise Sorbinsäure, p-Hydroxybenzoesäureester (zum Beispiel Niederalkylester), Benzoesäure, Natriumbenzoat, Trichlorisobutylalkohol, Phenol, Kresol, Benzethoniumchlorid und Formalinderivate in Betracht.

Die pharmazeutische und galenische Handhabung der erfindungsgemäßen Verbindungen erfolgt nach den üblichen Standardmethoden. Beispielsweise werden Wirkstoff(e) und Hilfs- beziehungsweise Trägerstoffe durch Rühren oder Homogenisieren (zum Beispiel mittels üblicher Mischgeräte) gut vermischt, wobei im allgemeinen bei Temperaturen

...

zwischen 20 und 80° C, vorzugsweise 20 bis 50° C, insbesondere bei Raumtemperatur gearbeitet wird. Im übrigen wird auf das folgende Standardwerk verwiesen: Sucker, Fuchs, Speiser, Pharmazeutische Technologie, Thieme-Verlag Stuttgart, 1978.

Die Applikation der Wirkstoffe beziehungsweise der Arzneimittel erfolgt auf die behaarte Kopfhaut.

Insbesondere ist auch der Zusatz anderer Arzneimittelwirkstoffe, vor allem von vasoconstrictorischen Mitteln möglich beziehungsweise günstig.

...

Der Gehalt der erfindungsgemässen aktiven Verbindungen in den Zubereitungen liegt im allgemeinen zwischen 20 und 100, vorzugsweise 40 und 80 mg pro Gramm Zubereitung. Dies gilt auch, wenn mehrere Verbindungen der Formel I in solchen Zubereitungen vorhanden sind.

Es ist auch möglich, die erfindungsgemässen Schwefelverbindungen in Suspension beispielsweise in Wasser, in einem Öl, einer Milch, einer Creme oder beispielsweise einem Gel (zum Beispiel hydrophile Gele) zu verwenden.

Es empfiehlt sich, den pharmazeutischen Zubereitungen, die die erfindungsgemässen Schwefelverbindungen enthalten, zusätzlich insbesondere vasokonstriktorisch wirkende Pharmaka zuzusetzen. Als solche Pharmaka kommen in Betracht: Cortison, Dexamethason, Betamethason, Triamcinolon, Hydrocortison. Der Anteil der vasokonstriktorischen Pharmaka zu den Schwefelverbindungen beträgt beispielsweise: 0,01 bis 0,1 Gewichtsteile vasokonstriktorisch wirkende Substanz zu 4 bis 10 Gewichtsteilen der erfindungsgemässen Schwefelverbindungen. Die vasokonstriktorisch wirkenden Substanzen können in den Lösungen, die die Thioverbindungen enthalten, in Konzentrationen von 0,01 bis 0,1 Gewichtsprozent vorzugsweise 0,02 bis 0,08 Gewichtsprozent vorliegen.

Der pH-Wert der erfindungsgemässen Zubereitungen kann beispielsweise zwischen 5,0 bis 9,3, vorzugsweise 8 - 9, insbesondere 8,6 liegen. Die Einstellung des pH-Wertes kann zum Beispiel mittels Phosphatpuffer ($NaH_2PO_4$/ $Na_2HPO_4$), Alkalihydroxiden (NaOH, $NaHCO_3$/$Na_2CO_3$ - Puffer) erfolgen.

...

Den erfindungsgemässen Zubereitungen können insbesondere auch übliche pharmazeutisch verwendbare Detergenzien (ionogene und nichtionogene) zugesetzt werden. Als derartige oberflächenaktive Substanzen kommen zum Beispiel in Betracht: Fettsäureester des Sorbitan und deren Äthoxylierungsprodukte (wie zum Beispiel Polyoxyethylen(20)-sorbitanmonolaurat, Polyoxyethylen (20)-sorbitanmonooleat), flüssige alkyl- und acylsubstituierte Polyadditionsorodukte des Ethylenoxids, Fettalkoholsulfate (zum Beispiel Natriumcetylstearylsulfat), Fettsäureester des Glycerins (zum Beispiel Glycerinmonostearat). Derartige oberflächenaktive Substanzen sind besipielsweise auch in H.P. Fiedler "Lexikon der Hilfsstoffe für Pharmazie, Kosmetik und angrenzende Gebiete", Verlag Cantor KG, Aulendorf in Württemberg 1971, Seite 132, 324 sowie 605-607 aufgeführt. Falls die erfindungsgemässen Thioverbindungen in Form einer Wasser-Öl-Emulsion angewendet werden, kommen beispielsweise als oberflächenaktive Stoffe insbesondere Fettsäureester des Glycerins und/oder Fettsäureester des Sorbitans in Betracht.

Die oberflächenaktiven Stoffe können als Einzelstoffe oder Gemische in den pharmazeutischen Zubereitungen zum Beispiel in einer Menge von 3 bis 10, vorzugsweise 5 bis 7 Gewichtsprozent vorliegen.

Die Verabreichung der erfindungsgemässen Schwefelverbindungen kann beispielsweise in Form von Gelees, Cremes oder in flüssiger Form erfolgen. Als flüssige Anwendungsformen kommen zum Beispiel in Frage: Ölige oder alkoholische beziehungsweise wässrige Lösungen sowie Suspensionen und Emulsionen, insbesondere Öl-Wasser-Emulsionen. Diese Zubereitungsformen enthalten beispielsweise zwischen 2 bis 10 Gewichtsprozent an aktiver Substanz.

...

Die Einzeldosis der erfindungsgemässen aktiven Komponenten kann beispielsweise bei Arzneiformen zur lokalen Applikation auf die Haut (zum Beispiel in Form von Lösungen, Lotionen, Emulsionen und so weiter) zwischen 50 und 100 ml, vorzugsweise 70 ml einer 2 - 10 %igen Lösung liegen.

Beispielsweise kann man hierbei wie folgt verfahren: Mit einer Lösung, die 2 - 10 Gewichtsprozente der Thioverbindung enthält, wird beispielsweise Verbandmull getränkt (50 - 100 ml, je nach der Kopfgröße, Quantität des Haares usw) und damit ein geeigneter Kopfverband (zum Beispiel nach Art des bekannten "Mitra Hippocratis") angelegt und schließlich mit einer undurchlässigen Schicht (am einfachsten zum Beispiel mit einer Badekappe) bedeckt.

Bei höheren Dosen des Zytostatikums wird man eine höhere Konzentration der Thioverbindung wählen und den Kopfverband häufig wechseln (dabei wird natürlich immer eine frische Lösung verwendet). Bei einer Gabe von zum Beispiel 50 bis 60 mg/Kg Ifosfamid täglich wird man die Thioverbindung auf die oben beschriebene Weise schon 1 Stunde vor Ifosfamid applizieren und die Behandlung bis zu 6 Stunden nach Injektion des Zytostatikums fortsetzen (Verbandwechsel zum Beispiel alle 2 Stunden).

Die akute Toxizität der erfindungsgemässen Verbindungen an der Maus (ausgedrückt durch die LD 50 mg/kg; Methode nach Miller und Tainter: Proc. Soc. Exper. Biol. a. Med. 57 (1944) 261) liegt beispielsweise bei intraperitonealer Applikation oberhalb 400 mg/kg.

...

Die Arzneimittel können in der Humanmedizin allein oder im Gemisch mit anderen pharmakologisch aktiven Stoffen verwendet werden.

Die Wirkung der erfindungsgemässen Schwefelverbindungen kann beispielsweise auch in tierexperimentellen Versuchen festgestellt werden. Ein besonders brauchbares tierexperimentelles Objekt zum Studium der durch Cytostatika induzierten Alopezie sind junge Ratten. In solchen Versuchen wurde gefunden, daß man bei perkutaner Applikation der erfindungsgemässen Verbindungen beziehungsweise einer Kombination von verschiedenen der erfindungsgemässen Verbindungen den nach der Applikation von Cytostatika, insbesondere von Oxazaphosphorinen (Ifosfamid, Cyclophosphamid) und aktivierten stabilisierten Oxazaphosphorinen bei 11 Tage alten Ratten regelmässig zu beobachtenden Haarausfall verhindern kann.

...

Die Wirkung ergibt sich beispielsweise aus folgenden Versuchen:

1. Elf Tage alten Ratten wird vor dem Versuch das Fell und die Haut durch Waschen mit Seife und warmem Wasser am Rücken gründlich entfettet. Ein passend gefaltetes Stück Mull wird anschließend mit einer Lösung von 2,64 Gewichtsprozent 2-Mercaptoessigsäure (neutralisiert mit 1 N NaOH) und 10 Gewichtsprozent Cystein in Wasser getränkt, auf die caudale Hälfte des Rückens appliziert und durch eine Lage von Gummiband und Tesaband abgedeckt. Dadurch entsteht ein flüssigkeitsdichter und luftundurchlässiger sogenannter Okklusivverband. Nach Erfahrungen der dermatologischen Therapie ist dadurch die Penetration der Wirkstoffe in die Haut besonders begünstigt. Den Verband läßt man vor Applikation des Cytostatikums 1,5 Stunden einwirken. Dann wird den Jungtieren das bekannte Cytostatikum "Ifosfamid" intraperitoneal verabreicht (100 mg/kg) und wie eingangs beschrieben die perkutane Behandlung mit den genannten Schwefelverbindungen über 2 weitere Stunden fortgeführt. Während bei den Kontrolltieren die "Ifosfamid"-Applikation zum totalen Haarausfall führt, bleiben bei den mit den Schwefelverbindungen perkutan behandelten Ratten die Haare an der caudalen Rückenhälfte erhalten.

2. Elf Tage alte Ratten werden 1,5 Stunden mit einer Lösung von 10 Gewichtsprozent Cysteaminhydrochlorid und 2,64 Gewichtsprozent 2-Mercaptoessigsäure (neutralisiert mit 1 N NaOH) in Wasser (wie unter 1. beschrieben) perkutan (caudale Rücken-

...

hälfte) behandelt. Wie unter 1. wird anschliessend "Ifosfamid" appliziert (100 mg/kg, intraperitoneal) und anschliessend über 2 weitere Stunden mit der obigen Lösung perkutan behandelt. Im Gegensatz zu den Kontrolltieren (totaler Haarausfall) bleibt bei den perkutan behandelten Tieren das Haar auf den entsprechenden Hautstellen erhalten.

Anstelle des Okklusivverbandes kann man bei den zuvor angegebenen Versuchen die Tiere auch mit der entsprechenden Lösung pinseln (Dauer der Behandlung vor und nach Applikation des Cytostatikums ist dieselbe). Dieses Verfahren führt zu denselben Ergebnissen wie das vorher erwähnte, ist jedoch leichter durchführbar. Dabei ist es unbedingt nötig, einen geeigneten Pinsel zu verwenden; bewährt hat sich zum Beispiel ein "Glattstrichpinsel" mit einem Streichkopf aus einem Spezialschaum. Dadurch ist jede Beschädigung der Haut der Jungtiere ausgeschlossen. Bei Verwendung der gewöhnlichen Pinsel ist dagegen eine Läsion der Epidermis durch Kunststoff- oder Naturborsten möglich.

Die wässrige Lösung von Cystein und Natriummercaptoacetat wurde wie folgt hergestellt: 2,64 g 2-Mercaptoessigsäure (zur Analyse) werden in 28 ml 1 N NaOH gelöst, dann werden zu dieser Lösung 10 g Cystein (für biochemische Zwecke) zugefügt und mit demineralisiertem Wasser auf 100 g aufgefüllt. Die Lösung soll gleich nach der Herstellung perkutan appliziert werden.

...

Beispiele für die Herstellung von galenischen Zubereitungen:

1. 3 g Natriummercaptoacetat (hergestellt durch Neutralisation von reiner 2-Mercaptoessigsäure mit 1 N NaOH) und 10 g Cystein werden in 100 ml Phosphat-Puffer (pH 8,0) gelöst.

2. 2 ml 2-Mercapto-essigsäure werden in etwas Wasser gelöst, mit 1 N NaOH auf pH 8,0 gebracht und mit Aqua dest. auf 100 ml aufgefüllt.

3. 5 g Maisstärke werden in 100 ml einer heißen Glycerin/$H_2O$-Mischung (40 Gewichtsprozent Glycerin) gelöst; nach Abkühlung auf 45° C werden 10 g Cystein und 2 - 3 g Natriumthioglycolat zugefügt und mit einem Glasstab gerührt, bis sich die Substanzen gelöst haben.

...

ASTA-Werke Aktiengesellschaft, Chemische Fabrik
Artur-Ladebeck-Straße 128-152, 4800 Bielefeld 14

Verwendung von Thioverbindungen zur Verhinderung des durch Cytostatika verursachten Haarausfalls

Patentansprüche:

1. Mittel zur Verhinderung der Cytostatika-Alopezie, dadurch gekennzeichnet, daß es als Wirkstoff mindestens eine Verbindung der allgemeinen Formel

$$Alk_1\text{-}S\text{-}Alk_2 \qquad I$$

enthält, worin $Alk_1$ einen Phenylrest, der gegebenenfalls durch eine Aminogruppe substituiert ist, einen $C_1$-$C_6$-Alkylrest, welcher einen Guanidinorest oder einen Guanidinorest mit einem, zwei oder drei $C_1$-$C_4$-Alkylresten enthält oder worin $Alk_1$ einen $C_1$-$C_6$-Alkylrest darstellt, welcher durch die Reste $R_1$, $R_2$ und $R_3$ substituiert ist, wobei die Reste $R_1$, $R_2$ und $R_3$ gleich oder verschieden sind und Wasserstoff, Hydroxygruppen, Mercaptogruppen, Sulfogruppen, Aminogruppen, $C_1$-$C_4$-Alkylaminogruppen, $C_1$-$C_4$-Dialkylaminogruppen, $C_2$-$C_6$-Alkanoylaminogruppen,

...

Carboxygruppen, $C_1$-$C_4$-Alkoxy-carbonylgruppen, Carboxymethylaminogruppen, Carboxymethylamino-carbonylgruppen, eine Phenylaminogruppe, die im Phenylrest gegebenenfalls eine Aminogruppe enthält oder eine Cycloalkylaminogruppe, bei welcher eine $CH_2$-Gruppe des Cycloalkylrestes auch durch ein Stickstoff- oder Sauerstoffatom ersetzt sein kann, bedeutet und $Alk_2$ Wasserstoff ist oder S-$Alk_1$ bedeutet; oder worin beide Reste $Alk_1$ und $Alk_2$ zusammen mit dem Schwefelatom einen gesättigten 5-Ring aus $CH_2$-Gliedern bilden, wobei ein Ringglied auch eine NH-Gruppe oder eine CO-Gruppe sein kann und dieser 5-Ring durch die Reste $R_5$, $R_6$ und $R_7$ substituiert ist, wobei die Reste $R_5$, $R_6$ und $R_7$ gleich oder verschieden sind und Wasserstoff, $C_1$-$C_4$-Alkylgruppen, Carboxygruppen, Aminogruppen oder $C_2$-$C_6$-Alkanoylaminogruppen sind; oder worin $Alk_1$ Wasserstoff oder ein $C_1$-$C_6$-Alkylrest ist, der gegebenenfalls eine Aminogruppe, eine Mono-$C_1$-$C_4$-alkylaminogruppe oder Di-$C_1$-$C_4$-alkylaminogruppe enthält und $Alk_2$ die Gruppe $-SO_3H$ oder die Gruppe $C(Y)-NR_8R_9$ bedeutet, worin Y die Gruppe $=NR_{10}$ oder ein Schwefelatom ist und die Reste $R_8$, $R_9$ und $R_{10}$ Wasserstoff oder $C_1$-$C_4$-Alkylreste sind und wobei einer der Reste $R_8$, $R_9$ oder $R_{10}$ auch ein Phenylrest oder ein Pyridylrest sein kann; oder daß das Mittel als Wirkstoff mindestens eine der folgenden Verbindungen enthält: 2-Mercapto-ethyl-phosphonsäure, Amidothiophosphorsäure, S-(2-Aminoethyl)-thiophosphorsäure, S-2-(3-Aminopropylamino)-ethyl-thiophosphorsäure, Guanylthioharnstoff, $\alpha$-Liponsäure, $\alpha$-Liponsäureamid, wobei die als Wirkstoffe in Frage kommenden Verbindungen auch in Form ihrer Salze mit physiologisch unbedenklichen Säuren beziehungsweise Kationen vorliegen können.

...

2. Mittel nach Anspruch 1, wobei in der Formel I $Alk_2$ Wasserstoff ist und $Alk_1$ ein $C_1$-$C_6$-Alkylrest ist, welcher eine Carboxygruppe oder eine Carb-$C_1$-$C_4$-alkoxygruppe enthält und zusätzlich in Nachbarstellung zu der Carboxy- oder Carbalkoxygruppe auch eine Aminogruppe oder eine $C_2$-$C_6$-Alkanoylaminogruppe enthalten kann.

3. Mittel nach Anspruch 1 und/oder Anspruch 2, dadurch gekennzeichnet, daß es den oder die Wirkstoffe zusammen mit üblichen physiologisch unbedenklichen Trägern, Hilfsstoffen und/oder Verdünnungsmitteln enthält.

4. Mittel nach einem oder mehreren der vorangegangenen Ansprüche, dadurch gekennzeichnet, daß die Wirkstoffe in Lösung vorliegen, wobei die Lösungsmittel Wasser, Ethanol oder Glycerin beziehungsweise Mischungen dieser Mittel sind.

5. Mittel nach einem oder mehreren der vorangegangenen Ansprüche, dadurch gekennzeichnet, daß außer einem oder mehreren der in Anspruch 1 und/oder Anspruch 2 genannten Wirkstoffe mindestens ein vasokonstriktorischer Stoff zugegegen ist.

6. Mittel nach einem oder mehreren der vorangegangenen Ansprüche, dadurch gekennzeichnet, daß die Wirkstoffe gemäß Anspruch 1 und/oder Anspruch 2 Cystein und/oder Mercaptoessigsäure sind.

...

7. Verwendung eines Wirkstoffes nach Anspruch 1 und/oder Anspruch 2 zur Verhinderung des durch Cytostatika verursachten Haarausfalls.

8. Verwendung eines Wirkstoffes nach Anspruch 1 und/oder Anspruch 2 zur Herstellung eines Mittels gegen den durch Cytostatika verursachten Haarausfall.

9. Verfahren zur Herstellung eines Mittels nach einem oder mehreren der vorangegangenen Ansprüche, dadurch gekennzeichnet,
daß man einen oder mehrere Wirkstoffe nach Anspruch 1 und/oder Anspruch 2 mit üblichen physiologisch unbedenklichen Träger- und/oder Hilfsstoffen und/oder Verdünnungsmitteln vermischt oder verarbeitet.

...